# EUROPÄISCHE PATENTSCHRIFT

(11) **EP 2 931 135 B1**
(45) Veröffentlichungstag und Bekanntmachung des Hinweises auf die Patenterteilung: **04.10.2017**
(21) Anmeldenummer: 13795428.5
(22) Anmeldetag: 12.11.2013
(51) Int. Cl.: A61B 17/00, A61F 2/04, A61B 17/12, A61M 25/10

(54) **BALLONKATHETER ZUR BEHANDLUNG ENTEROATMOSPHÄRISCHER FISTELN BEI OFFENEN ABDOMEN**
BALLOON CATHETER FOR TREATING ENTEROATMOSPHERIC FISTULAE IN AN OPEN ABDOMEN
CATHÉTER À BALLON POUR LE TRAITEMENT DE FISTULES ENTÉRO-ATMOSPHÉRIQUES EN ABDOMEN OUVERT

(30) Priorität: 12.12.2012 DE 102012024254
(43) Veröffentlichungstag der Anmeldung: 21.10.2015
(73) Patentinhaber: Adamidis, Georgios, 66482 Zweibrücken (DE)
(72) Erfinder: Adamidis, Georgios, 66482 Zweibrücken (DE)
(74) Vertreter: Patentanwälte Dr. Keller, Schwertfeger
(86) Internationale Anmeldenummer: PCT/EP2013/003402
(87) Internationale Veröffentlichungsnummer: WO 2014/090360

(56) Entgegenhaltungen:
- WO-A1-2013/119879
- DE-A1-102010 019 795
- US-A1- 2009 281 634

## Beschreibung

### Technisches Gebiet:

Die vorliegende Erfindung betrifft einen Ballonkatheter zur Behandlung enteroatmosphärischer Fisteln bei offenen Abdomen.

### Stand der Technik:

Enterokutane Fisteln entstehen als Folge entzündlicher Darmerkrankungen (z.B. Colitis ulcerosa, Morbus Crohn) oder als Komplikation bei chirurgischen Eingriffen am Magen-Darm-Trakt. Dabei stellen enterokutane Fisteln einen nicht natürlichen Verbindungsgang zwischen dem Darm und der Haut der Baudecke dar. Bei solchen offenen Abdomen steigt die Mortalität der Betroffenen durch die Ausbildung einer intestinalen Fistel auf 40 bis 60 % an (Schein MS, Decker GA. Postoperative external alimentary tract fistulas. Am J Surg. 1191; 161: 435-438).

Häufig lösen intestinale Fisteln Infektionen, wie z.B. eine Peritonitis aus, die den Schweregrad der Erkrankung drastisch erhöhen. Mit Hilfe der Enterostomie wird seit einiger Zeit versucht, die Ausscheidungen des Darms über eine chirurgisch herbeigeführte Öffnung eines Darmteils durch die Bauchwand auszuleiten. Hierbei sind Ausleitungen in unterschiedlichen Darmteilen möglich, beispielsweise im Ileum, Caecum, Colon sigmoideum oder Colon transversum.

Im Gegensatz zu enterokutanen Fisteln werden Fisteln, die im frei liegenden Bereich des offenen Abdomens enden, als "enteroatmosphärisch" bezeichnet. Die bislang bekannten Ableitungssysteme, Klebe- und Nahttechniken sowie Defektdeckungen stellen keine verlässliche Behandlungsoption dar. Die Patienten sterben vielfach an den Komplikationen. Mit der Einführung der Vakuumtherapie wurde in Morbidität und Mortalität bei der Behandlung eines offenen Abdomens verbessert (van Hensbroek PB, Wind J, Dijkgraaf MGW et al: Temporary closure of the open abdomen; A systematic review on delayed primary fascial closure in patients with an open abdomen. World J Surg. 2009; 33: 199-207 / Wild T, Stortecky S, Stremitzer S et al: Abdominal Dressing - ein neuer Standard in der Behandlung des offenen Abdomens infolge sekundärer Peritonitis. Zentralabl Chir. 2006; 131: 111-114). Die Vakuumtherapie hat jedoch den Nachteil, dass aufgrund des wirkenden Unterdrucks die Fistel und damit auch der Darmwanddurchbruch häufig vergrößert werden. Dies führt dazu, dass zusätzliche Ausscheidungen nach Außen gelangen. Aufgrund des austretenden, oft dickflüssigen Sekrets kommt es zusätzlich zur Verstopfung des bei der Vakuumtherapie eingesetzten Saugschwammes, wodurch die Wirksamkeit der Saugung abnimmt. Durch die austretenden Ausscheidungen drohen Kontaminationen, Infektionen und Wundheilstörungen. Die Vakuumtherapie kann somit keine zufriedenstellende Behandlungsmethode darstellen.

Zwar bestehen Bestrebungen, die Fistel vom Unterdrucksystem zu trennen und ein Verkleben des Schwammes zu verhindern, jedoch sind solche Maßnahmen deutlich aufwändiger (Goverman J, Yelon JA, Platz JJ et al. The "Fistula VAC", a technique for management of enterocutanous fistulae arising within the open abdomen: Report of 5 cases. J Trauma, 2006; 60: 428-431 / Brünner W. Walzel G. Modifiziertes V.A.C.-System in der Behandlung enteraler Fisteln bei offenen Abdomen: Innovation - Indikation - technische Grundlagen, ZfW., 2009: No. A: 56-58).

Eine alternative Lösung sieht die Verwendung eines Fisteladapters vor, der aus einem Zylinder aus flexiblem Material besteht, um die mechanische Irritation der Viszera zu minimieren (Jannasch Olof, Hans Lippert, Jörg Tautenhahn: "Ein neuer Adapter zur Versorgung von enteratmosphärischen Fisteln beim offenen Abdomen"; Pharmetra, 2010). Der Fisteladapter wird in eine entsprechend zugeschnittene Öffnung im Polyurethanschwamm eingesetzt. Anschließend wird der Schwamm inklusive Fisteladapter auf die Wunde aufgebracht. Der PU-Schwamm kann vollständig mit einer Polyurethanfolie überklebt werden. Anschließend wird ein zweiteiliges Stomaset aufgeklebt. Durch den anliegenden Unterdruck wird der Fisteladapter in der gewählten Position sicher gehalten. Zwar werden bei der Verwendung eines Fisteladapters die Probleme der Vakuumtherapie verringert. Jedoch stellt auch ein Fisteladapter keine verlässliche Methode bei der Behandlung enteratmosphärischer Fisteln dar.

Daneben gab es Versuche, Ernährungs- oder Urinkatheter zur Behandlung enteratmosphärischer Fisteln einzusetzen (Everson AR, Fischer JE.; Current management of enterocutaneous fistula. J Gastrointest Surg. 2006; 10: 455-464 / Medeiros AC, Aires-Neto T, Marchini JS et al. Treatment of postoperative enterocutaneous fistulas by high-pressure vacuum with normal oral diet. Dig Surg. 2004; 21: 401-405). Allerdings scheitern solche Katheter an dem oft zähflüssigen Sekret, der regelmäßig zu Verstopfungen des Katheterlumens geführt hat. Häufig konnte man bei geblockten Kathetern sogar eine Größenzunahme der Fisteln beobachten.

Eine weitere Vorrichtung zur Sekretableitung sieht einen Saugaufsatz vor, der mit der Unterseite auf die Fistel aufgebracht wird (Layton B, DuBose J. Nichols S et al. Pacifiying the open abdomen with concomitant intestinal fistula; a novel approach. Am J Surg. 2010; 199: e48-e50).

Aufgrund der speziellen Problematik bei der Behandlung enteroatmosphärischer Fisteln und den Anforderungen bei der Ableitung von zähflüssigem Sekret durch die Bauchdecke sind daher die bislang bekannten Kathetersysteme, wie sie beispielsweise in der DE 60 2005 005 567 T2, DE 11 2006 002 272 T5 oder der DE 11 2008 003 106 T5 beschrieben sind, nicht geeignet.

In der DE 10 2010 019 795 A1, die als nächstliegender Stand der Technik betrachtet wird, wird ein Doppelballonkathetersystem beschrieben, dass zur Abdichtung von Punktionsstellen oder Öffnungen in Körperhöhlen, Hohlorganen oder bei perkutanen Drainagen in Säugetieren zur Anwendung kommen soll. Bei der Behandlung enteroatmosphärischer Fisteln wäre jedoch ein solches Ballonkathetersystem nicht geeignet, da die beiden Ballons einen zu großen Druck auf die Darmwand ausüben wurden, was zu Nekrosen in der Fistelregion führen würde. In der EP 1 022 033 A1 ist ein Katheter zur intravaskulären Verbindung zweier Gefäßabschnitte beschrieben, der jedoch nicht mit Ballons fixiert ist und bei dem zudem die Leitung zur Fluidzufuhr sowie die mit Gefäßflüssigkeit durchströmbare Verbindungsleitung unterschiedliche Volumen aufweisen.

### Darstellung der Erfindung:

Vor diesem Hintergrund ist es Aufgabe der vorliegenden Erfindung, einen verbesserten oder alternativen Katheter bereit zu stellen, der zur wirksamen Behandlung enteroatmosphärischer Fisteln bei offenen Abdomen geeignet ist.

Diese Aufgabe wird gelöst durch einen Ballonkatheter mit den Merkmalen des Anspruchs 1.

Bevorzugte Ausführungsformen finden sich in den Unteransprüchen wieder.

Der erfindungsgemäße Doppel-Ballonkatheter besteht aus einem innen hohlen Einführungsrohr mit einem vorzugsweise in etwa zylindrischen Querschnitt. Das Einführungsrohr besitzt ein Lumen zur Ableitung von Darmsekreten bzw. zur Einleitung von Spülflüssigkeit oder Medikamenten in den Darm. An dem Einführungsrohr ist ein erster, distaler Ballon angeordnet, der den Schaft des Einführungsrohrs vollständig umgibt. Darunter ist ein zweiter, proximaler Ballon angeordnet. Beide Ballons dienen der Abdichtung der Fistelöffnung.

Der distale Ballon und der proximale Ballon werden über wenigstens eine Injektionsleitung mit einem Füllmedium (z.B. Luft oder NaCl-Lösung) befüllt, wodurch die Ballonhaut aufgeblasen wird. Am Fußende des Einführungsrohres ist ein quer, d.h. im rechten Winkel dazu angeordnetes Passagerohr ausgebildet, dessen Lumen mit dem Lumen des Einführungsrohres verbunden ist und das in den Darmabschnitt in der Fistelregion einführbar ist. Vorzugsweise bilden das Einführungsrohr und das Passagerohr ein T-Stück. Bevorzugt stellt das T-Stück ein einzelnes Bauteil dar, jedoch können Einführungsrohr und Passagerohr auch getrennte Einheiten sein, die bedarfsweise zusammengesetzt werden. Die beiden Ballons sind vorzugsweise fest mit dem Einführungsrohr verbunden.

Das Passagerohr besteht vorzugsweise aus einem elastischen, biegsamen Material, so dass es durch den Operateur leicht über die Fistelöffnung in das Innere des Darmabschnittes eingeführt werden kann, ohne die Fistelöffnung unnötig aufzuweiten. Dieser Vorgang verläuft bei dem erfindungsgemäßen Katheter äußerst schonend. Hierfür wird zunächst ein Ende des Passagerohrs über die Fistelöffnung in den Darmkanal geführt, anschließend wird das kontralaterale Ende des Passagerohrs in entgegen gesetzter Richtung in den Darmkanal eingeführt. Das Einführungsrohr wird über die Fistelöffnung und das offene Abdomen nach außen geführt.

Die Fistelregion ist der Bereich des Körpers des Betroffenen, in dem sich das offene Abdomen (d.h. die offene Bauchdecke) und der Darmdurchbruch befinden. Die Fistelöffnung bezeichnet den nach Außen offenen Durchbruch des Darms sowie gegebenenfalls das darüber liegende Gewebe.

Durch das im Darmkanal angeordnete Passagerohr werden der Darminhalt und die Sekrete an der Fistelregion, insbesondere der Fistelöffnung, vorbei geführt. Die üblichen auftretenden Komplikationen, wie sie oben beschrieben wurden, werden deutlich vermindert oder gar ganz vermieden. In einer bevorzugten Ausführungsform besitzt das Passagerohr des Ballon-Kathetersystems einen Außendurchmesser, der in etwa dem Innendurchmesser des Darmabschnittes in der Fistelregion entspricht. Eine zusätzliche Fixierung des Passagerohrs im Darmkanal ist beispielsweise mit einem zusätzlichen Ballon möglich, der das Passagerohr umschließt. Eine solche Ausführungsform ist weiter unten beschrieben.

Über wenigstens eine Injektionsleitung werden nach erfolgter Platzierung des Katheters der distale und der proximale Ballon mit Füllmedium inflatiert, wodurch das Ballonvolumen jeweils zunimmt. In einer bevorzugten Ausführungsform besitzt jeder Ballon eine eigene Injektionsleitung, wodurch die einzelnen Ballonvolumina individuell einstellbar sind. Im eingesetzten Zustand dichtet der distale Ballon die Fistelöffnung von außen ab, während der proximale Ballon den Durchbruch von innen abdichtet. Durch die entgegenwirkenden Anpressdrucke der beiden Ballons wird der Darmdurchbruch bzw. die Fistelöffnung wirksam abgedichtet. Zwischen den beiden Ballons befinden sich vorzugsweise ein oder mehrere Saugplättchen (zum Beispiel ein Schwamm), um die Fistel zusätzlich abzudichten, Feuchtigkeit aufzunehmen und den Druck der beiden Ballons an der Fistelöffnung abzudämpfen.

Je nach Bauform der Ballons besteht die Gefahr, dass die Ballons zu stark in die Fistelöffnung gedrückt werden und diese dadurch unnötig aufweiten. Um dies zu vermeiden, sind der distale Ballon und/oder der proximale Ballon vorzugsweise keulenförmig ausgestaltet. Dadurch wirken die Anpressdrucke in einem Radius um die Fistelöffnung an den Keulenenden, nicht jedoch an der Fistelöffnung selbst. Zudem ist eine flüssigkeitsfeste Abdichtung gewährleistet. Der Druck wird von der eigentlichen Fistelöffnung in den Randbereich der Fistelregion verlagert.

In einer bevorzugten Ausführungsform sind die beiden Ballons entlang des Einführungsrohrs beweglich. In einer alternativen Ausführungsform sind die Ballons fest mit dem Einführungsrohr verbunden. Die bewegliche Ausführungsform hat den Vorteil, dass die beiden Ballons entsprechend der Wandstärke des Darmkanals, des Durchmessers der Fistelöffnung und dem umliegenden Gewebe angepasst werden können. Eine Anpassung kann zudem über das Füllvolumen der einzelnen Ballons erfolgen.

Vorzugsweise ist das Volumen des distalen Ballons größer als das Volumen des proximalen Ballons, um dem Druck der Darmpassage wirksam entgegen zu wirken.

In einer alternativen Ausführungsform ist an dem Passagerohr des erfindungsgemäßen Ballonkatheters wenigstens ein zusätzlicher aufblasbarer Ballon angeordnet, um das Passagerohr im Darmkanal zu fixieren. Vorzugsweise befindet sich an den beiden Enden des Passagerohrs jeweils ein Ballon. Damit ist eine sichere Fixierung des Systems innerhalb des Darmkanals gewährleistet. Der Darminhalt wird durch das Passagerohr an der Fistelöffnung vorbei geleitet.

Der erfindungsgemäße Ballonkatheter kann zur Herstellung eines Medizinproduktes für die Behandlung von enteroatmosphärischen Fisteln bei offenen Abdomen eingesetzt werden. Das Medizinprodukt besteht beispielsweise aus dem erfindungsgemäßen Ballonkatheter, einer Gebrauchsanleitung und gegebenenfalls einer Spritze zur Befüllung der Ballons über die Injektionsleitungen mit Füllmedium. Ferner können dem Medizinprodukt Ersatz-Saugplättchen beigefügt sein.

### Kurze Beschreibung der Zeichnungen:

Die Erfindung wird in den nachfolgenden beiden Zeichnungen näher erläutert.

Es zeigen
- Fig. 1: eine Ausführungsform des erfindungsgemäßen Ballonkatheters,
- Fig. 2: ein Anwendungsbeispiel des erfindungsgemäßen Ballonkatheters bei der Behandlung enteroatmosphärischer Fisteln,
- Fig. 3: eine weitere Ausführungsform des erfindungsgemäßen Ballonkatheters mit zusätzlichen Ballons am Passagerohr.

### Wege zur Ausführung der Erfindung und gewerbliche Verwertbarkeit:

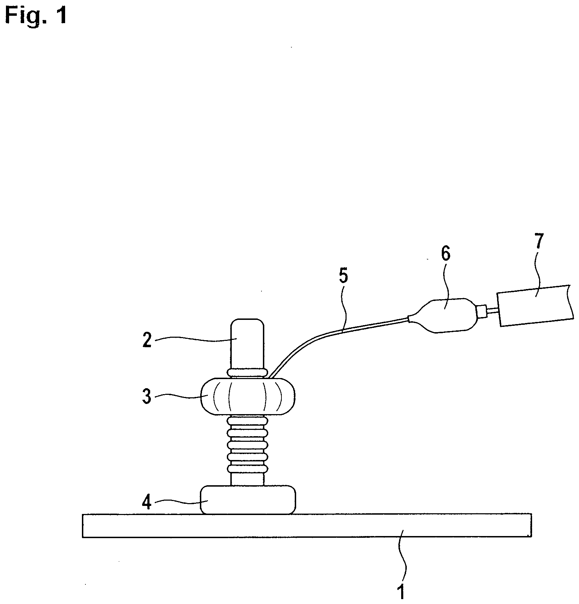

In Fig. 1 ist der Grundaufbau des erfindungsgemäßen Ballonkatheters gezeigt. Ein Einführungsrohr 2 ist mit einem quer dazu angeordneten Passagerohr 1 verbunden. Das Einführungsrohr 2 und das Passagerohr 1 bilden ein T-Stück. Um den Schaft des Einführungsrohrs 2 ist ein distaler Ballon 3 angeordnet. Darunter ist ein ebenfalls am Schaft ausgebildeter proximaler Ballon 4 angeordnet. Zur Inflation der beiden Ballons 3, 4 ist wenigstens eine Injektionsleitung 5 vorgesehen. Zum Befüllen der Ballons 3, 4 kann beispielsweise Kochsalzlösung oder Luft eingesetzt werden. Das Füllmedium wird vorzugsweise über eine Spritze 7 zugeführt. Ein Ventil 6 verhindert einen Rücklauf der Injektionsflüssigkeit.

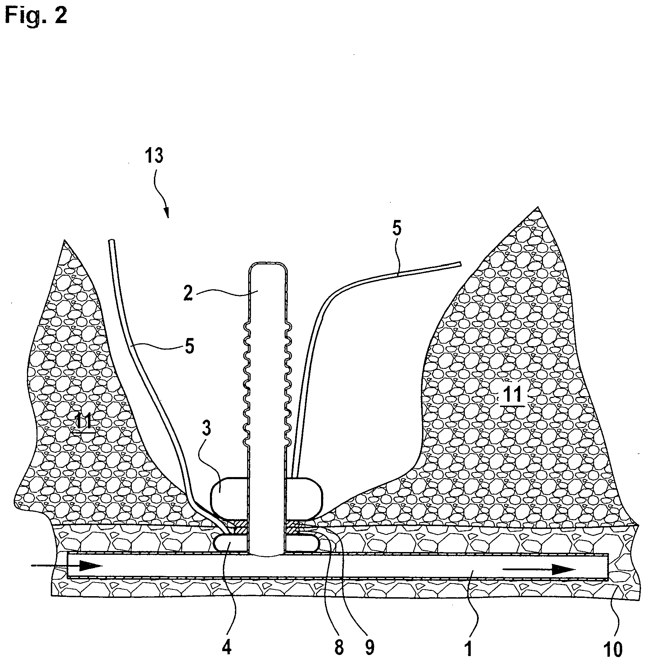

In Fig. 2 ist ein Anwendungsbeispiel des Ballonkatheters der Ausführungsform gemäß Fig. 1 gezeigt. Veranschaulicht ist ein offenes Abdomen 13 mit umliegendem Gewebe 11 sowie einem Darmkanal 10. Durch die Fistelöffnung wird zunächst ein laterales Ende des Passagerohrs 1 in den Darmkanal 10 eingeführt. Danach wird das kontralaterale Ende des Passagerohrs 1 in entgegen gesetzter Richtung über die Fistelöffnung eingesetzt. Dabei sitzt der proximale Ballon 4 im Darmlumen und drückt gegen die Innenwand, während der distale Ballon 3 von der Außenseite gegen das Gewebe bzw. die Darmwand drückt. Über getrennte Injektionsleitungen 5 werden die beiden Ballons 3, 4 mit Füllmedium (Luft) befüllt. Vorzugsweise sind die beiden Ballons 3, 4 keulenförmig ausgestaltet, so dass der Anpressdruck in der Fistelregion in einem größeren Radius um die Fistelöffnung verteilt wird, wodurch ein Aufweiten der Fistelöffnung vermindert bzw. verhindert wird. Zwischen den beiden Ballons 3, 4 befinden sich ein oberes Saugplättchen 9 und ein unteres Saugplättchen 8.

Der Darminhalt kann durch das Passagerohr 1 passieren, ohne dass zähflüssiges Sekret über das Einführungsrohr 2 austritt.

Über das Lumen des Einführungsrohrs 2 können (beispielsweise von oben) Spülflüssigkeit oder Medikamente eingebracht werden, wodurch beispielsweise ein Verstopfen des Einführungsrohres 2 oder des Passagerohrs 1 verhindert oder eine Reinigung ermöglicht wird. Als Spülflüssigkeit kann beispielsweise Kochsalzlösung eingesetzt werden.

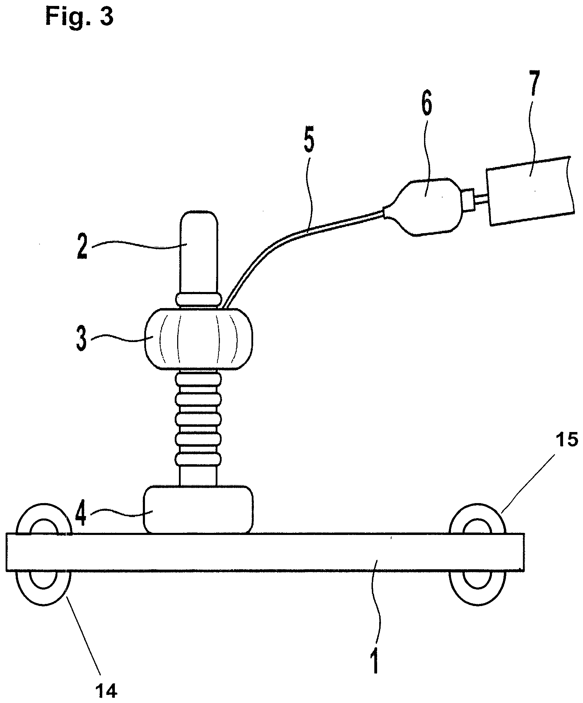

In Fig. 3 ist eine weitere Ausführungsform des erfindungsgemäßen Ballonkatheters gezeigt, bei dem zwei zusätzliche Ballons 14, 15 am Passagerohr 1 angeordnet sind. Das Passagerohr 1 besteht aus einem elastischen, biegsamen Material, z.B. einem PU-Kunststoff. Die beiden endseitig angeordneten Ballons 14, 15 werden nach Einführung des Passagerohrs 1 in den Darmkanal mit Füllmedium gefüllt und aufgeblasen. Dadurch wird das System in dem Darmkanal fixiert. Sofern die Ballons 14, 15 den Darm in dieser Region ausfüllen, wird der Darminhalt durch das Passagerohr 1 geleitet.

Der erfindungsgemäße Katheter ist leicht in der Handhabung, kostengünstig herzustellen und vermeidet die bekannten Komplikationen.

## Patentansprüche

1. Ballonkatheter zur Behandlung enteroatmosphärischer Fisteln bei offenen Abdomen (13), bestehend aus einem Einführungsrohr (2) mit einem um den Schaft angeordneten distalen Ballon (3) und einem darunter angeordneten proximalen Ballon (4) zur Abdichtung der Fistel, wenigstens einer Injektionsleitung (5) zur Befüllung des distalen und proximalen Ballons (3, 4) mit Füllmedium, **gekennzeichnet durch** ein zum Einführungsrohr (2) an dessen Fußende quer dazu angeordnetes Passagerohr (1), dessen Lumen mit dem Lumen des Einführungsrohrs (2) verbunden ist und in den Darmkanal einführbar ist.

2. Ballonkatheter nach Anspruch 1, **dadurch gekennzeichnet, dass** der distale Ballon (3) und/oder der proximale Ballon (4) keulenförmig ausgestaltet ist.

3. Ballonkatheter nach Anspruch 1 oder 2, **dadurch gekennzeichnet, dass** das Passagerohr (1) einen Außendurchmesser aufweist, der in etwa dem Innendurchmesser des Darmabschnittes in der Fistelregion entspricht.

4. Ballonkatheter nach einem der vorhergehenden Ansprüche, **dadurch gekennzeichnet, dass** das Passagerohr (1) aus einem elastischen, biegsamen Material besteht.

5. Ballonkatheter nach einem der vorhergehenden Ansprüche, **dadurch gekennzeichnet, dass** das Einführungsrohr (2) und das Passagerohr (1) zu einem T-Stück ausgebildet sind.

6. Ballonkatheter nach einem der vorhergehenden Ansprüche, **dadurch gekennzeichnet, dass** zwischen dem distalen Ballon (3) und dem proximalen Ballon (4) ein oder mehrere Saugplättchen (8, 9) angeordnet sind.

7. Ballonkatheter nach einem der vorhergehenden Ansprüche, **dadurch gekennzeichnet, dass** der distale Ballon (3) im inflatierten Zustand ein größeres Volumen besitzt, als der proximale Ballon (4).

8. Ballonkatheter nach einem der vorhergehenden Ansprüche, **dadurch gekennzeichnet, dass** der distale Ballon (3) und der proximale Ballon (4) mit einer eigenen Injektionsleitung (5) verbunden sind.

9. Ballonkatheter nach einem der vorhergehenden Ansprüche, **dadurch gekennzeichnet, dass** am dem Passagerohr (1) wenigstens ein zusätzlicher Ballon (14, 15) angeordnet ist.

## Claims

1. Balloon catheter for treating enteroatmospheric fistulae in an open abdomen (13), consisting of an insertion tube (2) having a distal balloon (3) arranged around the shaft and a proximal balloon (4) arranged thereunder for sealing off the fistula, and having at least one injection line (5) for filling the distal and the proximal balloons (3, 4) with filling medium, **characterised by**
a passage tube (1) arranged transversely to the insertion tube (2) at the bottom end thereof, the lumen of which passage tube is connected to the lumen of the insertion tube (2) and can be inserted into the intestinal canal.

2. Balloon catheter according to claim 1, **characterised in that** the distal balloon (3) and/or the proximal balloon (4) is/are shaped like a club.

3. Balloon catheter according to either claim 1 or claim 2, **characterised in that** the passage tube (1) has an outside diameter that corresponds approximately to the inside diameter of the intestinal section in the fistula region.

4. Balloon catheter according to any of the preceding claims, **characterised in that** the passage tube (1) consists of a resilient, flexible material.

5. Balloon catheter according to any of the preceding claims, **characterised in that** the insertion tube (2) and the passage tube (1) are designed to form a T-piece.

6. Balloon catheter according to any of the preceding claims, **characterised in that** one or more small suction plates (8, 9) are arranged between the distal balloon (3) and the proximal balloon (4).

7. Balloon catheter according to any of the preceding claims, **characterised in that** the distal balloon (3) has a greater volume in the inflated state than the proximal balloon (4).

8. Balloon catheter according to any of the preceding claims, **characterised in that** the distal balloon (3) and the proximal balloon (4) are each connected to an individual injection line (5).

9. Balloon catheter according to any of the preceding claims, **characterised in that** at least one additional balloon (14, 15) is arranged on the passage tube (1).

## Revendications

1. Cathéter à ballon destiné au traitement des fistules entéro-atmosphériques avec l'abdomen (13) ouvert, composé d'un tube d'introduction (2) pourvu d'un ballon distal (3) disposé autour de la tige et d'un ballon proximal (4) disposé au-dessous de celle-ci pour réaliser l'étanchéité de la fistule, au moins une conduite d'injection (5) destinée à remplir les ballons (3, 4) distal et proximal d'un fluide de remplissage, **caractérisé par** un tube de passage (1), disposé transversalement par rapport au tube d'introduction (2) à son extrémité antérieure, dont le lumen est relié au lumen du tube d'introduction (2) et lequel peut être inséré dans le canal intestinal.

2. Cathéter à ballon selon la revendication 1, **caractérisé en ce que** le ballon distal (3) et/ou le ballon proximal (4) sont réalisés en forme de massue.

3. Cathéter à ballon selon la revendication 1 ou 2, **caractérisé en ce que** le tube de passage (1) présente un diamètre extérieur qui correspond approximativement au diamètre intérieur de la portion d'intestin dans la région de la fistule.

4. Cathéter à ballon selon l'une des revendications précédentes, **caractérisé en ce que** le tube de passage (1) se compose d'un matériau élastique flexible.

5. Cathéter à ballon selon l'une des revendications précédentes, **caractérisé en ce que** le tube d'introduction (2) et le tube de passage (1) sont réalisés sous la forme d'une pièce en T.

6. Cathéter à ballon selon l'une des revendications précédentes, **caractérisé en ce qu'**une ou plusieurs plaquettes d'aspiration (8, 9) sont disposées entre le ballon distal (3) et le ballon proximal (4).

7. Cathéter à ballon selon l'une des revendications précédentes, **caractérisé en ce que** le ballon distal (3) à l'état gonflé possède un volume supérieur à celui du ballon proximal (4).

8. Cathéter à ballon selon l'une des revendications précédentes, **caractérisé en ce que** le ballon distal (3) et le ballon proximal (4) sont reliés par une conduite d'injection (5).

9. Cathéter à ballon selon l'une des revendications précédentes, **caractérisé en ce qu'**au moins un ballon supplémentaire (14, 15) est disposé sur le tube de passage (1).
